# FASCICULE DE BREVET EUROPEEN

(11) **EP 0 878 166 B1**
(45) Date de publication et mention de la délivrance du brevet: **14.12.2005**
(21) Numéro de dépôt: 98401181.7
(22) Date de dépôt: 15.05.1998
(51) Int. Cl.: A61B 5/20, F16K 11/085

(54) **Dispositif pour examen urodynamique et éléments le composant**
Vorrichtung zur urodynamischen Untersuchung und seine Komponenten
Device for urodynamic examination and its components

(30) Priorité: 15.05.1997 FR 9706003
(43) Date de publication de la demande: 18.11.1998
(73) Titulaire: Laboratoires Ercelab Vermed, 27470 Serquigny (FR)
(72) Inventeur: Lourdel, Philippe, 60660 Cires-Les-Mellos (FR); Marchetti, Pascal, F-27410 Beaumesnil (FR)
(74) Mandataire: Texier, Christian

(56) Documents cités:
- FR-A- 2 119 257
- FR-A- 2 684 902
- US-A- 4 063 548
- US-A- 4 474 149
- US-A- 5 331 548
- US-A- 5 385 563

## Description

L'invention se rapporte au domaine médical des examens urologiques du type urodynamique et vise en particulier un dispositif permettant de pratiquer cet examen ainsi que les éléments le composant. Un tel dispositif est par exemple connu de US-A- 5 385 563 qui décrit un dispositif comprenant les caractéristiques définies dans le préambule de la revendication 1.

L'examen urodynamique vise à étudier le comportement dynamique du système vésico-sphinctérien inclus dans l'enceinte abdominale. Il est mené à l'aide d'une machine spécialisée qui enregistre les pressions mesurées dans la vessie, l'urètre et l'abdomen.

Dans les systèmes à prise de pression à eau, on dispose d'une sonde qui mesure les pressions vésicales et urétrales et une sonde pour la prise de pression abdominale.

Le principe de mesure de la pression vésicale consiste à remplir la vessie avec de l'eau et à mesurer en même temps la pression intravésicale.

La prise de pression urétrale consiste à perfuser un liquide à débit constant au travers d'un orifice latéral d'une sonde introduite dans l'urètre et à mesurer les variations de pression en amont en fonction de la position de la sonde le long de l'urètre. Ce type de mesure a fait notamment l'objet d'une étude complète par BROWN et WICKHAM qui est rapportée dans l'article intitulé : « The urethral pressure profile » et paru dans la revue The British Journal of Urology, avec les références 1969 - 41-211.216.

Dans les deux cas, l'élément de transmission de pression étant la colonne d'eau, une purge soignée du dispositif est nécessaire. En outre, pour la mesure de la pression urétrale qui est du type à mesure de pression par perfusion de liquide, le débit doit être très précis - par exemple 2 ml/min. - jusque des contre-pressions élevées, supérieures à 250 cm de colonne d'eau.

Pour la pression abdominale on mesure la pression à l'intérieur de l'ampoule rectale.

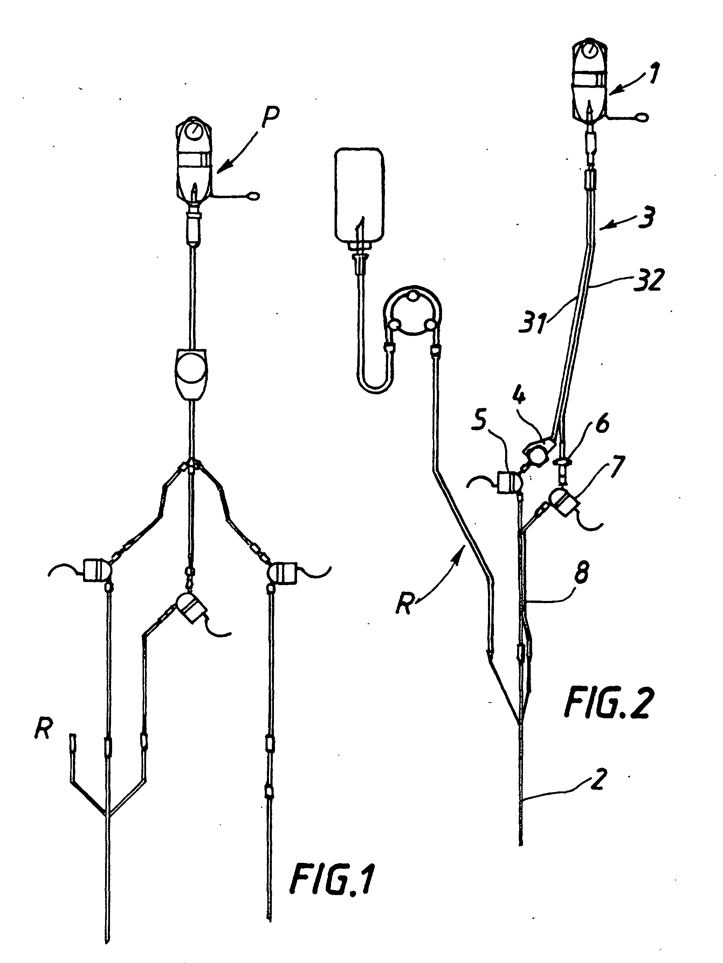

La figure 1 représente un dispositif utilisé communément pour pratiquer cet examen.

Une poche d'eau soumise à une pression P communique par une ligne de raccordement avec 3 capteurs de pression vésicale, urétrale et abdominale. Un régulateur de débit, du type par exemple clamp à roulette, est placé en amont des capteurs.

Des raccords individuels relient les capteurs aux sondes.

On connaît deux méthodes pour l'alimentation en fluide. La méthode gravitationnelle consiste à suspendre la poche à une certaine hauteur, 2 m par exemple. Cette méthode est peu onéreuse et la purge des conduits est facile à réaliser par simple ouverture du clamp.

Toutefois le débit est peu précis et est variable en fonction des contre-pressions générées dans l'urètre.

Pour remédier à ce problème, on a proposé de mettre la poche sous pression au moyen d'un manchon associé à un manomètre. Toutefois, avec ce système le débit est encore peu précis. En particulier, il existe des pertes de charge communes qui perturbent les mesures. Une variation de perte de charge sur une voie influence la perte de charge sur les autres voies.

On a alors proposé de placer dans les voies des limiteurs de débit fixes, tels que des tubes de faible diamètre formant des résistances hydrauliques, qui assurent un débit plus stable, mais dans ce cas le temps de purge est très long.

En raison de la communication entre les voies, une contamination est toujours possible entre la voie abdominale et la voie vésicale et urétrale.

Un autre aspect de la prise de pression en urodynamique est la capacité du système de mesure à enregistrer aussi bien les pressions stables ou à variations très lentes, que les variations rapides (lors d'une toux par exemple).

Ainsi afin d'assurer une bonne transmission des pressions entre la sonde et le capteur, les prolongateurs sont conçus pour apporter le minimum de perturbations en temps de réponse.

Les prolongateurs sont nécessaires quand l'examen doit être effectué dans des positions différentes du patient : couchée, assise. Ils évitent également la contamination des capteurs par reflux de liquide ou un contact direct de la sonde.

Le problème rencontré avec ces prolongateurs est qu'ils rendent le système sensible aux parasites et artefacts. En effet ils transmettent aussi bien les pressions de la sonde que les mouvements et artefacts qui leur sont appliqués. Ceci est d'autant plus gênant que les perturbations enregistrées sont dans la bande de fréquences de mesures effectuées. Par exemple le balancement d'un prolongateur peut être interprété comme une contraction vésicale.

Enfin quand on utilise une sonde de prise de pression urétrale à deux voies. Celle-ci comprend deux éléments tubulaires enfilés l'un dans l'autre. Leurs extrémités proximales ont une certaine liberté de mouvement l'une par rapport à l'autre. Des mesures de pression parasites peuvent être enregistrées en raison de ce mouvement relatif.

On résout ce problème en proposant, conformément à l'invention un dispositif pour examen urodynamique comprenant une source de fluide de perfusion, au moins deux capteurs de pression et une sonde avec au moins une lumière pour prise de pression vésicale et une lumière pour prise de pression urétrale, un premier moyen de raccordement tubulaire entre la source de fluides et les capteurs et un deuxième moyen de raccordement tubulaire entre les capteurs et la sonde, caractérisé en ce qu'au moins l'un des deux moyens est constitué de deux éléments tubulaires solidaires l'un de l'autre sur la majeure partie de leur longueur.

En effet, cette disposition permet de supprimer les écarts de pression entre les deux moyens de raccordement tubulaire ne ressortant pas des mesures effectuées. En outre, en ce qui concerne le premier moyen de raccordement entre la source de fluide et les capteurs, on supprime toute perte de charge commune. Les variations de pression sur l'une des voies n'aura pas d'effet sur l'autre.

Un autre avantage de cette solution est de proposer un dispositif dont le montage est plus simple.

Selon une autre caractéristique, les deux moyens de raccordement sont constitués chacun de deux éléments tubulaires solidaires entre eux. Ce mode permet de réaliser simplement par collage ou autre moyen équivalent le raccordement double.

Selon une autre caractéristique le premier moyen de raccordement comprend au moins un limiteur de débit. Un tel limiteur de débit peut être réalisé au moyen d'un tube capillaire convenablement calibré.

Selon une autre caractéristique le premier moyen de raccordement comprend un commutateur de débit constitué par un limiteur de débit, un conduit bipassant ledit limiteur de débit et un sélecteur pour sélectionner soit le passage du fluide par le limiteur de débit soit le passage par le conduit de bipasse, soit l'interruption de l'écoulement du fluide.

Selon une autre caractéristique le premier moyen comprend au moins un robinet sur l'un des organes tubulaires.

Selon une autre caractéristique la sonde de mesure comprend deux éléments tubulaires solidaires entre eux sur toute leur longueur.

Selon une autre caractéristique le dispositif d'examen urodynamique comprend un moyen indépendant de mesure de la pression abdominale.

L'invention concerne également un moyen de raccordement entre une source de pression et au moins deux capteurs dudit dispositif pour examen urodynamique. Il est caractérisé en ce qu'il comprend deux éléments tubulaires solidaires entre eux sur la majeure partie de leur longueur avec chacun un premier connecteur pour un raccordement à une poche de fluide et un deuxième connecteur pour un raccordement à l'un des connecteurs des capteurs.

Selon une autre caractéristique il comprend un robinet monté sur au moins l'un des éléments tubulaires.

Selon une autre caractéristique il comprend un commutateur de débit constitué par un limiteur de débit, un conduit bipassant celui-ci et un organe sélecteur pour choisir le passage soit à travers le limiteur soit le bipasse.

L'invention concerne également un moyen de raccordement entre deux capteurs de pression et une sonde de mesure de pression dudit dispositif pour examen urodynamique caractérisé en ce qu'il est constitué de deux éléments tubulaires solidaires entre eux sur la majeure partie de leur longueur et terminés chacun par des organes de connexion.

Un mode de réalisation de l'invention concerne également un commutateur de débit pour être monté notamment sur un dispositif d'examen urodynamique caractérisé en ce qu'il comprend un boîtier à 3 voies, un limiteur de débit monté en dérivation sur une voie et débouchant sur la seconde voie, et un sélecteur rotatif à 3 voies permettant de choisir soit un écoulement à débit limité soit un écoulement sans limitation au travers dudit commutateur soit une position fermée.

Un mode de réalisation de l'invention concerne également un moyen de commutation de débit pour être monté sur un système de mesure de pression par perfusion notamment sur un dispositif selon la revendication 1 caractérisé en ce qu'il comprend deux conduits parallèles dont l'un est pourvu d'un limiteur de débit

L'invention sera mieux comprise à la lecture de la description d'un mode de réalisation non limitatif accompagnée de dessins sur lesquels :
La figure 1 représente un mode de réalisation conforme à l'art antérieur.
La figure 2 représente un dispositif conforme à l'invention.
La figure 3 représente un mode de réalisation du premier moyen de raccordement conforme à l'invention.
La figure 4 représente un mode de réalisation du second moyen de raccordement conforme à l'invention.
Les figures 5a, 5b, 5c représentent le schéma de principe d'un limiteur de débit associé à un sélecteur conforme à l'invention.
La figures 6 représente un second mode de réalisation d'un limiteur de débit associé à un sélecteur conforme à l'invention.
La figure 7 représente une sonde à deux voies conforme à l'invention.

Sur la figure 2, on voit représenté un dispositif selon l'invention. Une poche (1) de liquide alimente une sonde (2) de prise de pression vésicale et urétrale. Il s'agit d'une sonde à 3 voies connue en soi : Une voie pour la pression vésicale, une voie pour la pression urétrale et une voie communiquant avec une ligne séparée (R) pour le remplissage de la vessie.

La poche ou source de perfusion (1) communique avec un premier moyen de raccordement tubulaire (3) qui est constitué de deux tubes (31) et (32) solidaires sur la majeure partie de leur longueur. Le tube (31) comprend à son autre extrémité un commutateur de débit (4), et se termine par un connecteur par lequel il est relié à un premier capteur de pression (5). Le deuxième tube (32) comprend à son autre extrémité un robinet (6), et se termine par un connecteur par lequel il est relié à un deuxième capteur de pression (7).

Un deuxième moyen de raccordement tubulaire (8) relie chacun des capteurs (5) et (7) à la sonde (2). Ce deuxième moyen est constitué de deux tubes (81) et (82) mettant chacun en communication un capteur à une lumière, respectivement vésicale et urétrale, de la sonde (2).

Les deux tubes (81) et (82) sont solidaires sur la majeure partie de leur longueur.

Les capteurs (5 et 7) comprennent de façon connue une chambre communiquant par des connecteurs appropriés avec les éléments tubulaires des deux moyens de raccordement (3) et (8). La chambre de fluide des capteurs comprend une paroi dont le déplacement résultant de la pression exercée par le fluide est transformée en un signal électrique qui est dirigé vers un enregistreur non représenté.

Le dispositif fonctionne de la façon suivante.

Avant la mise en place de la sonde dans l'urètre, on ouvre le commutateur du moyen (4) et le robinet (6) de façon à purger la ligne de toute bulle d'air. On introduit ensuite la sonde jusque dans la vessie et on ferme les deux lignes par (4) et (6).

On peut alors procéder à la mesure de la pression vésicale en remplissant progressivement la vessie au moyen de la ligne de remplissage. La pression du fluide est mesurée par le capteur (7).

Pour l'examen de l'urètre, on place le commutateur de débit (4) en position de limitation de débit. Ainsi un faible débit de fluide est perfusé dans la ligne jusqu'à l'urètre. La valeur de la pression du fluide relevée par le capteur (5) est fonction de la résistance à l'écoulement exercée par la paroi de l'urètre sur l'orifice latéral de la sonde.

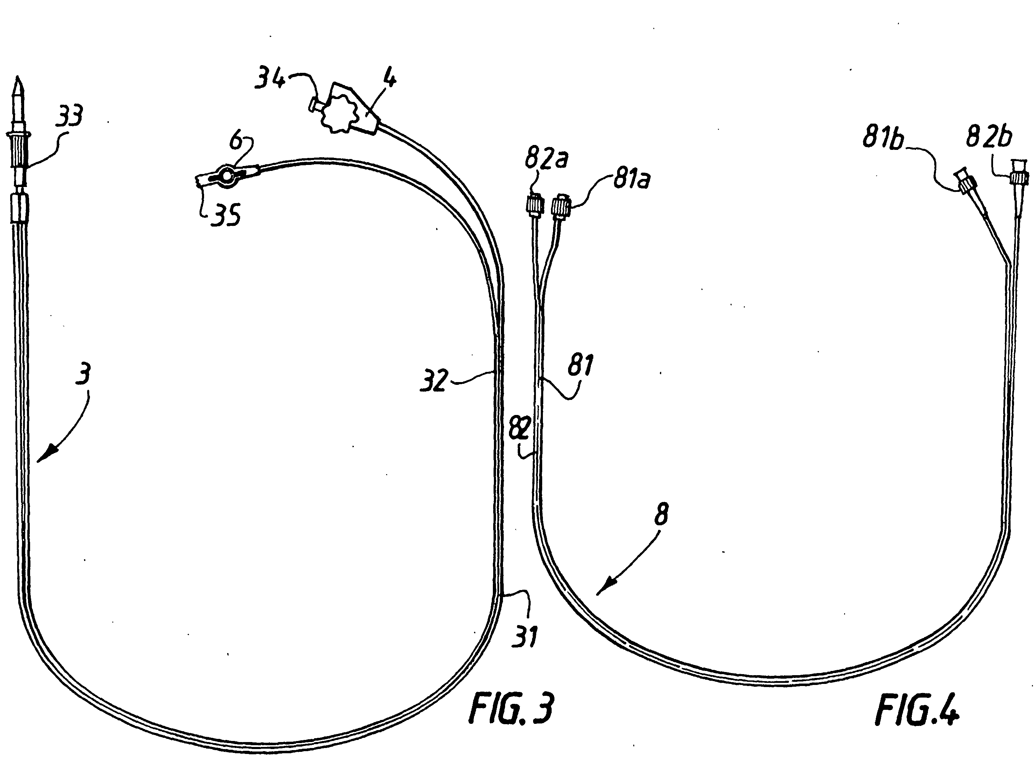

On a représenté sur les figures 3 à 6 les différents éléments du dispositif. La figure 3 montre le premier moyen de raccordement (3). Il comprend deux tubes (31) et (32) pourvus ensemble à une extrémité d'un percuteur (33) que l'on raccorde à la source de fluide (1). A son autre extrémité, le tube (31) est relié à un commutateur de débit (4) décrit plus en détail plus loin. Ce moyen (4) est lui-même pourvu d'un moyen de connexion (34) permettant le branchement à un capteur de pression (5).

Le tube (32) comprend à son autre extrémité un robinet (6) pouvant prendre deux positions : une ouverte, l'autre fermée. Le robinet se termine lui-même par un moyen de connexion (35). Ainsi que cela apparaît sur la figure, les deux tubes 31 et 32 sont collés l'un à l'autre sur la majeure partie de leur longueur.

Seules les extrémités sont libres pour faciliter leur raccordement respectif aux capteurs.

La figure 4 montre le deuxième moyen de raccordement tubulaire (8). Il est constitué de deux tubes (81) et (82) collés l'un à l'autre sur la majeure partie de leur longueur et terminés chacun par des connecteurs (81a, 81b) et (82a, 82b) permettant le branchement aux capteurs (5) et (7) d'un côté et aux deux lumières de la sonde (2) de l'autre.

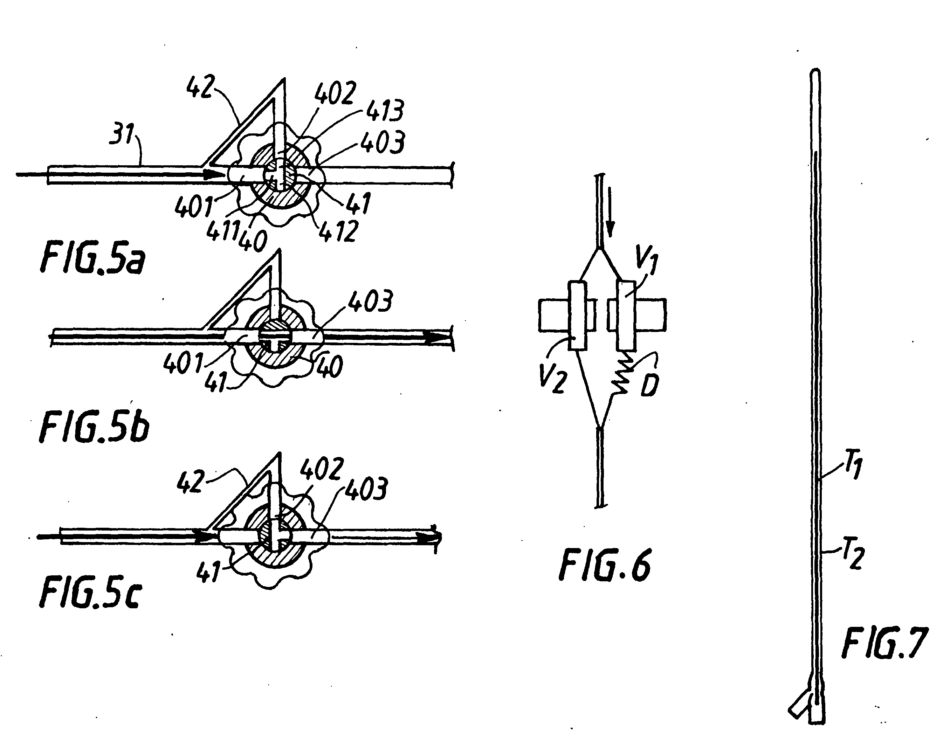

La figure 5 montre le schéma de principe d'un commutateur de débit (4) conforme à l'invention. Il comprend un boîtier (40) avec un sélecteur (41) rotatif

La partie fixe, ou boîtier (40), est à 3 voies (401 - 402 - 403). La voie (401) communique avec le tube (31), et la voie (403) communique avec un connecteur pour être branché au capteur. La voie (402) débouche entre les deux premières.

Un sélecteur (41) à 3 voies également (411 - 412 - 413) est monté à rotation dans le boîtier.

Un tube capillaire (42), formant résistance hydraulique, est branché en dérivation sur la voie (401) et débouche dans la voie (402).

Les figures 5a, 5b, 5c montrent trois positions du sélecteur. Sur la figure 5a, le sélecteur (41) est en position fermée; la voie (403) est obturée. Aucun liquide ne s'écoule au travers du commutateur. Sur la figure 5b le sélecteur est en position de purge. Les voies (412 et 413) du sélecteur mettent en communication les voies (401 et 403) du boîtier. Le liquide s'écoule sans résistance depuis le tube (31) vers le capteur. Le débit est par exemple de 20 à 30 ml/min. Sur la figure 5c le sélecteur est en position de limitation de débit. Les voies (411) et(412) mettent en communication les voies (402 et 403) du boîtier. Le liquide s'écoule par le tube capillaire 42. Le débit est compris entre 0,5 et 5 ml/min.

Selon un autre mode de réalisation du commutateur de débit représenté sur la figure 6 on a remplacé le dispositif à sélecteur rotatif par un ensemble à deux vannes montées sur deux conduits parallèles dont l'un comprend un limiteur de débit (D) capillaire. Une vanne (V1) est en série avec le limiteur de débit (D). Les deux vannes (V1 et V2) peuvent être mises en position ouverte ou fermée indépendamment l'une de l'autre. Ainsi selon que l'on ouvre l'une ou l'autre vanne, on autorise soit un débit de purge soit un débit de perfusion.

Sur la figure 7 on a représenté un mode de réalisation de sonde à deux voies où les moyens de connexion sont solidaires l'un de l'autre éliminant de ce fait les mouvements relatifs entre eux. Dans ce type de sonde, les deux tubes (T1, T2) constituant chacun une voie de prise de pression sont enfilés l'un dans l'autre sur toute leur longueur jusqu'aux moyens de connexion. Par rapport à l'ancien type de sonde tous les mouvements sont simultanés. On élimine ainsi les parasites de pression sur l'un des capteurs par rapport à l'autre.

Conformément à une autre caractéristique de l'invention, le dispositif complet pour réaliser un bilan urodynamique est constitué d'éléments qui protègent le patient de contaminations éventuelles soit d'un patient à l'autre soit de la voie anale vers la voie vésicale, tout en permettant une réutilisation dans certaines limites de certains éléments. Dans ce but, les éléments sont répartis en un groupe dit à usage journalier et un groupe dit à usage unique.

Les éléments du groupe à usage unique comprennent les sondes : sonde anale, non représentée, pour la mesure de la pression abdominale comme mesure de référence et sonde urétrale. On comprend dans ces éléments à usage unique, le moyen de raccordement tubulaire double 8 mais aussi un moyen de raccordement tubulaire à valve, non représenté, entre le sonde urétrale et la ligne séparée R assurant le remplissage de la vessie. Les éléments du groupe à usage unique sont changés après chaque bilan effectué sur un patient.

Les éléments du groupe journalier peuvent n'être changés qu'après chaque séance de bilan sur plusieurs patients dans la mesure où ils ne risquent pas d'être contaminés au cours de cette séance. Il s'agit du groupe constitué par les capteurs, la source de perfusion et leur moyen de raccordement, la ligne de remplissage de la vessie et la voie de prise de pression abdominale

La longueur des moyens de raccordement tubulaire entre la sonde et les capteurs est déterminée de façon que, en usage normal par le manipulateur, en cas de chute de pression du système d'alimentation de la voie de perfusion (le cas le plus défavorable étant une surpression de 100 cm H2O dans la vessie), cette longueur soit suffisante pour protéger les capteurs d'un retour de liquide donc de contamination éventuelle. Par exemple, on dimensionne le raccord de façon à laisser 15 minute de réaction au praticien en cas d'une surpression de 100 cm H2O dans la vessie, avec un reflux se propageant à une vitesse de 3cm par 30 secondes (50 cm de raccord plus 40 cm de sonde).

Ainsi si le praticien déconnecte la sonde des raccords pour vidanger la vessie, il risque uniquement de contaminer l'extrémité des raccords et non les capteurs. Cet aménagement permet donc de classer les capteurs et a fortiori les lignes pour prise de pression urétrale, en amont des capteurs, dans le groupe d'usage journalier : environ 5 bilans sur une journée. La notion d'usage journalier vient de la constatation qu'il existe des risques de contamination du système par le manipulateur, les intervenants externes (pour l'entretien ou le SAV par exemple) ou bien en raison de la stagnation de l'eau dans les tubulures. Ainsi la maîtrise des risques en dehors d'une période d'utilisation contrôlée par le manipulateur devient impossible. Il faut donc par mesure de sécurité l'ensemble des éléments formant le groupe à usage journalier soit démonté à la fin de chaque séance de bilan qui s'échelonne sur une journée.

Dans le même but on prévoit un moyen de raccordement supplémentaire entre la ligne de remplissage et la voie de remplissage de la sonde pour bilan urodynamique. La valve à pour fonction d'empêcher tout retour de liquide vers la ligne. Lors de la déconnexion de la sonde pour vidanger la vessie, la valve oblige le praticien à déconnecter la sonde de la valve et ainsi l'empêche de contaminer l'extrémité de la ligne de remplissage qui peut être alors réutilisée dans le cadre d'un usage journalier.

Par ailleurs on sépare complètement la voie anale comprenant notamment un système de mesure par pression à air de la voie vésicale.

L'invention ne se limite pas au mode de réalisation représenté mais englobe assurément toute les variantes à la portée de l'homme du métier.

## Revendications

1. Dispositif pour examen urodynamique comprenant une source (1) de fluide de perfusion, au moins deux capteurs de pression (5, 7) et une sonde (2) avec au moins une lumière pour prise de pression vésicale et une lumière pour prise de pression urétrale, un premier moyen (3) de raccordement tubulaire entre la source (1) de fluide et les capteurs (5, 7) et un deuxième moyen (8) de raccordement tubulaire entre les capteurs (5, 7) et la sonde (2), **caractérisé en ce qu'**au moins l'un des deux moyens (3, 8) est constitue de deux éléments tubulaires (31, 32; 81 82) solidaires l'un de l'autre sur la majeure partie de leur longueur.

2. Dispositif selon la revendication 1 **caractérisé en ce que** les deux moyens de raccordement sont constitués chacun de deux éléments tubulaires solidaires entre eux.

3. Dispositif selon l'une des revendications précédentes, **caractérisé en ce que** la sonde de mesure (2) comprend deux éléments tubulaires solidaires entre eux sur toute leur longueur.

4. Dispositif selon l'une des revendications précédentes **caractérisé en ce qu'**il comprend un moyen indépendant de mesure de la pression abdominale.

5. Dispositif selon l'une des revendications précédentes, **caractérisé en ce que** le premier moyen (3) de raccordement comprend au moins un limiteur de débit.

6. Dispositif selon la revendication 5, **caractérisé en ce que** le premier moyen (3) de raccordement comprend un commutateur de débit constitué par un limiteur de débit, un conduit bipassant ledit limiteur et un sélecteur pour sélectionner soit le passage du fluide par le limiteur de débit soit le passage par le conduit de bipasse soit interrompant l'écoulement de fluide.

7. Dispositif pour examen urodynamique selon la revendication 5, **caractérisé en ce qu'**il comprend en outre un moyen de commutation de débit (4) pour être monté entre le premier (3) et le deuxième (8) moyens de raccordement, comprenant un boîtier à 3 voies, le limiteur de débit étant monté en dérivation sur une voie et débouchant sur la seconde voie, la première et la seconde voies étant respectivement connectées avec le premier et le deuxième moyens de raccordement (6 et 8), et un sélecteur rotatif à 3 voies permettant de choisir soit un écoulement à débit limité soit un écoulement sans limitation de débit soit une position fermée.

8. Dispositif pour examen urodynamique selon la revendication 5, **caractérisé en ce qu'**il comprend en outre un moyen de commutation de débit pour être monté entre le premier et le deuxième moyens de raccordement (3 et 8), comprenant deux conduits parallèles dont l'un est pourvu du limiteur de débit.

9. Dispositif selon l'une des revendications précédentes **caractérisé en ce que** le premier moyen (3) comprend au moins un robinet (6) sur l'un des éléments tubulaires.

10. Dispositif pour examen urodynamique selon l'une des revendications précédentes comprenant une source de fluide avec ligne séparée pour le remplissage de la vessie **caractérisé en ce qu'**il comprend un moyen de raccordement avec valve entre ladite ligne de remplissage et la sonde urétrale.

11. Dispositif pour examen urodynamique selon l'une des revendications précédentes **caractérisé en ce que** la longueur et le diamètre des seconds moyens de raccordement sont déterminés de façon à empêcher, en usage normal, la contamination des capteurs par reflux de liquide.

12. Dispositif pour examen urodynamique selon l'une des revendications précédentes **caractérisé en ce qu'**il est composé d'un premier groupe d'éléments à usage journalier constitué des capteurs, de la ligne de mesure de pression urétrale et de la ligne de remplissage, et d'un deuxième groupe d'éléments à usage unique constitué de la sonde urétrale avec ses moyens de raccordement aux capteurs et à la ligne de remplissage et le cas échéant d'une sonde anale pour la mesure de la pression abdominale.

13. Moyen de raccordement entre une source de pression (1) et au moins deux capteurs (5, 7) du dispositif selon l'une des revendications 1 à 12, **caractérisé en ce qu'**il comprend deux éléments tubulaires (31, 32) solidaires entre eux sur la majeure partie de leur longueur avec un premier connecteur pour un raccordement a une poche de fluide et un deuxième connecteur pour un raccordement aux connecteurs des capteurs.

14. Moyen de raccordement selon la revendication 13, **caractérisé en ce qu'**il comprend un robinet (6) monté sur au moins l'un des éléments tubulaires.

15. Moyen de raccordement selon la revendication 13 ou 14 **caractérisé en ce qu'**il comprend un commutateur de débit.

16. Moyen de raccordement entre deux capteurs (5, 7) de pression et une sonde (2) de mesure de pression du dispositif selon l'une des revendications 1 à 12, **caractérisé en ce qu'**il est constitué de deux éléments tubulaires (81, 82) solidaires entre eux sur la majeure partie de leur longueur et terminés chacun par des organes de connexion à une sonde de mesure.

## Patentansprüche

1. Vorrichtung zur urodynamischen Untersuchung, die eine Infusions flüssigkeitsquelle (1) wenigstens umfasst: zwei Druckmeßfühler (5, 7) und eine Sonde (2) mit wenigstens einem Kanal zur Aufnahme des Blasendrucks und einem Kanal zur Aufnahme des Harnröhrendrucks, ein erstes Mittel (3) zur rohrförmigen Verbindung zwischen der Fluidquelle (1) und den Meßfühlern (5, 7) und ein zweites Mittel (8) zur rohrförmigen Verbindung zwischen den Meßfühlern (5, 7) und der Sonde (2), **dadurch gekennzeichnet, daß** wenigstens eines der beiden Mittel (3, 8) von zwei rohrförmigen Elementen (31, 32; 81, 82) gebildet ist, die über den größten Teil ihrer Länge fest miteinander verbunden sind.

2. Vorrichtung nach Anspruch 1, **dadurch gekennzeichnet, daß** die beiden Verbindungsmittel von jeweils zwei rohrförmigen Elementen gebildet sind, die fest miteinander verbunden sind.

3. Vorrichtung nach einem der vorhergehenden Ansprüche, **dadurch gekennzeichnet, daß** die Meßsonde (2) zwei rohrförmige Elemente umfaßt, die über ihre gesamte Länge fest miteinander verbunden sind.

4. Vorrichtung nach einem der vorhergehenden Ansprüche, **dadurch gekennzeichnet, daß** sie ein unabhängiges Mittel zur Messung des Abdominaldrucks umfaßt.

5. Vorrichtung nach einem der vorhergehenden Ansprüche, **dadurch gekennzeichnet, daß** das erste Verbindungsmittel (3) wenigstens einen Durchflußbegrenzer umfaßt.

6. Vorrichtung nach Anspruch 5, **dadurch gekennzeichnet, daß** das erste Verbindungsmittel (3) einen Durchflußschalter umfaßt, der gebildet wird von einem Durchflüßbegrenzer, einer Leitung, die den genannten Begrenzer umgeht, und einer Wähleinrichtung, um den Durchgang der Flüssigkeit durch den Begrenzer, oder den Durchgang durch die Umgehungsleitung, oder das Unterbrechen des Ausströmens der Flüssigkeit zu wählen.

7. Vorrichtung zur urodynamischen Untersuchung nach Anspruch 5, **dadurch gekennzeichnet, daß** sie außerdem ein Mittel zur Durchflußschaltung (4) zum Einbau zwischen dem ersten (3) und dem zweiten (8) Verbindungsmittel umfaßt, das ein Dreiwegegehäuse umfaßt, wobei der Durchflußbegrenzer von einem Weg abzweigt und in den zweiten Weg einmündet, der erste und der zweite Weg mit dem ersten bzw. dem zweiten Verbindungsmittel (6 und 8) verbunden ist und es ein Dreiwege-Drehwähler gestattet, ein Strömen mit begrenztem Durchfluß oder ein Strömen ohne Durchflußbegrenzung oder eine geschlossene Position zu wählen.

8. Vorrichtung zur urodynamischen Untersuchung nach Anspruch 5, **dadurch gekennzeichnet, daß** sie außerdem ein Mittel zur Durchflußschaltung zum Einbau zwischen dem ersten und dem zweiten Verbindungsmittel (3 und 8) umfaßt, das zwei parallele Leitungen umfaßt, von denen die eine mit dem Durchflußbegrenzer versehen ist.

9. Vorrichtung nach einem der vorhergehenden Ansprüche, **dadurch gekennzeichnet, daß** das erste Mittel (3) wenigstens einen Absperrhahn (6) an einem der rohrförmigen Elemente umfaßt.

10. Vorrichtung zur urodynamischen Untersuchung nach einem der vorhergehenden Ansprüche, die eine Flüssigkeitsquelle mit getrennter Leitung zum Auffüllen der Blase umfaßt, **dadurch gekennzeichnet, daß** sie ein Verbindungsmittel mit Ventil zwischen der genannten Auffüllleitung und der Harnröhrensonde umfaßt.

11. Vorrichtung zur urodynamischen Untersuchung nach einem der vorhergehenden Ansprüche, **dadurch gekennzeichnet, daß** die Länge und der Durchmesser der zweiten Verbindungsmittel derart bestimmt sind, daß im Normalgebrauch die Kontamination der Meßfühler durch einen Flüssigkeits-Rückfluß verhindert ist.

12. Vorrichtung zur urodynamischen Untersuchung nach einem der vorhergehenden Ansprüche, **dadurch gekennzeichnet, daß** sie aufgebaut ist aus einer ersten Gruppe von Elementen zum täglichen Gebrauch, die aus den Meßfühlern, der Meßleitung des Harnröhrendrucks und der Auffüllleitung gebildet ist, und einer zweiten Gruppe von Elementen zum einmaligen Gebrauch, die von der Harnröhrensonde mit ihren Mitteln zur Verbindung mit den Meßfühlern und der Auffüllleitung sowie gegebenenfalls von einer Analsonde zur Messung des Abdominals gebildet ist.

13. Mittel zur Verbindung zwischen einer Druckquelle (1) und wenigstens zwei Meßfühlern (5, 7) der Vorrichtung nach einem der Ansprüche 1 bis 12, **dadurch gekennzeichnet, daß** es zwei rohrförmige Elemente (31, 32) umfaßt, die über den größten Teil ihrer Länge miteinander fest verbunden sind, mit einem ersten Anschlußteil für eine Verbindung mit einem Flüssigkeitsbeutel und einem zweiten Anschlußteil zur Verbindung mit den Anschlüssen der Meßfühler.

14. Verbindungsmittel nach Anspruch 13, **dadurch gekennzeichnet, daß** es wenigstens einen Absperrhahn (6) umfaßt, der an wenigstens einem der rohrförmigen Elemente angebracht ist.

15. Verbindungsmittel nach Anspruch 13 oder 14, **dadurch gekennzeichnet, daß** es einen Durchflußschalter umfaßt.

16. Mittel zur Verbindung zwischen zwei Druckmeßfühlern (5, 7) und einer Druckmeßsonde (2) der Vorrichtung nach einem der Ansprüche 1 bis 12, **dadurch gekennzeichnet, daß** es von zwei rohrförmigen Elementen (81, 82) gebildet ist, die über den größten Teil ihrer Länge fest untereinander verbunden sind und jeweils durch Organe des Anschlusses an einer Meßsonde enden.

## Claims

1. Device for urodynamic examination, comprising a source (1) of perfusion fluid, at least two pressure sensors (5, 7), and a probe (2) with at least one lumen for tapping bladder pressure and one lumen for tapping urethral pressure, a first tubular connecting means (3) between the fluid source (1) and the sensors (5, 7), and a second tubular connecting means (8) between the sensors (5, 7) and the probe (2), **characterized in that** at least one of the two means (3, 8) is composed of two tubular elements (31, 32; 81, 82) joined integrally to one another along most of their length.

2. Device according to Claim 1, **characterized in that** the two connecting means are each composed of two tubular elements joined integrally to one another.

3. Device according to either of the preceding claims, **characterized in that** the measurement probe (2) comprises two tubular elements joined integrally to one another along their entire length.

4. Device according to one of the preceding claims, **characterized in that** it comprises an independent means for measurement of the abdominal pressure.

5. Device according to one of the preceding claims, **characterized in that** the first connecting means (3) comprises at least one flow rate limiter.

6. Device according to Claim 5, **characterized in that** the first connecting means (3) comprises a flow switch formed by a flow rate limiter, a conduit bypassing said limiter, and a selector for selecting either passage of the fluid through the flow rate limiter or passage through the bypass conduit, or interruption of the flow of fluid.

7. Device for urodynamic examination according to Claim 5, **characterized in that** it additionally comprises a flow-switching means (4) to be fitted between the first (3) and second (8) connecting means, comprising a housing with three paths, the flow rate limiter being mounted as a branch on one path and opening into the second path, the first and second paths being respectively connected to the first and second connecting means (6 and 8), and a three-path rotary selector making it possible to choose either a flow at a limited flow rate or a flow without limitation of the flow rate, or a closed position.

8. Device for urodynamic examination according to Claim 5, **characterized in that** it additionally comprises a flow-switching means to be fitted between the first and second connecting means (3 and 8), comprising two parallel conduits, one of which is provided with the flow rate limiter.

9. Device according to one of the preceding claims, **characterized in that** the first means (3) comprises at least one tap (6) on one of the tubular elements.

10. Device for urodynamic examination according to one of the preceding claims, comprising a source of fluid with separate line for filling the bladder, **characterized in that** it comprises a connecting means with valve between said filling line and the urethral probe.

11. Device for urodynamic examination according to one of the preceding claims, **characterized in that** the length and the diameter of the second connecting means are determined so as to prevent, in normal use, contamination of the sensors by reverse flow of liquid.

12. Device for urodynamic examination according to one of the preceding claims, **characterized in that** it is composed of a first group of elements for daily use consisting of the sensors, the line for measuring urethral pressure and the filling line, and a second group of disposable elements consisting of the urethral probe with its means of connection to the sensors and to the filling line and, where appropriate, of an anal probe for measurement of the abdominal pressure.

13. Connecting means between a pressure source (1) and at least two sensors (5, 7) of the device according to one of Claims 1 to 12, **characterized in that** it comprises two tubular elements (31, 32) joined integrally to one another along most of their length, with a first connector for attachment to a bag of fluid, and a second connector for attachment to the connectors of the sensors.

14. Connecting means according to Claim 13, **characterized in that** it comprises a tap (6) fitted on at least one of the tubular elements.

15. Connecting means according to Claim 13 or 14,
**characterized in that** it comprises a flow switch.

16. Connecting means between two pressure sensors (5, 7) and a pressure measurement probe (2) of the device according to one of Claims 1 to 12, **characterized in that** it is composed of two tubular elements (81, 82) which are joined integrally to one another along most of their length and which each terminate with members for connection to a measurement probe.
